# EUROPÄISCHE PATENTSCHRIFT

(11) **EP 2 731 923 B1**
(45) Veröffentlichungstag und Bekanntmachung des Hinweises auf die Patenterteilung: **09.09.2015**
(21) Anmeldenummer: 12733089.2
(22) Anmeldetag: 03.07.2012
(51) Int. Cl.: C07C 6/06, C07C 13/24

(54) **VERFAHREN ZUR HERSTELLUNG VON CYCLOHEPTEN**
PROCESS FOR PREPARING CYCLOHEPTENE
PROCÉDÉ DE PRODUCTION DE CYCLOHEPTÈNE

(30) Priorität: 12.07.2011 EP 11173656
(43) Veröffentlichungstag der Anmeldung: 21.05.2014
(73) Patentinhaber: BASF SE, 67056 Ludwigshafen (DE)
(72) Erfinder: TELES, Joaquim Henrique, 67165 Waldsee (DE); LIMBACH, Michael, 67551 Worms (DE); DEHN, Richard, 67061 Ludwigshafen (DE); DEUERLEIN, Stephan, 67061 Ludwigshafen (DE); DANZ, Manuel, 68723 Plankstadt (DE)
(86) Internationale Anmeldenummer: PCT/EP2012/062944
(87) Internationale Veröffentlichungsnummer: WO 2013/007561

(56) Entgegenhaltungen:
- US-A1- 2009 258 866
- I. W. ASHWORTH ET AL: "On the relationship between structure and reaction rate in olefin ring-closing metathesis.", CHEMICAL COMMUNICATIONS, Bd. 46, 2010, Seiten 7145-7147, XP002684624, SEINSTITUTE OF INORGANIC AND PHYSICAL CHEMISTRY, STOCKHOLM, ISSN: 0366-5607
- DENMARK S E ET AL: "Sequential ring-closing metathesis/Pd-catalyzed, Si-assisted cross-coupling reactions: general synthesis of highly substituted unsaturated alcohols and medium-sized rings containing a 1,3-cis-cis diene unit", TETRAHEDRON, ELSEVIER SCIENCE PUBLISHERS, AMSTERDAM, NL, Bd. 60, Nr. 43, 18. Oktober 2004 (2004-10-18), Seiten 9695-9708, XP004573966, ISSN: 0040-4020, DOI: 10.1016/J.TET.2004.06.149
- V. S.C. YEH ET AL: "Synthesis and structural activity relationship of 11beta-HSD1 inhibitors with novel adamantane replacements", BIOORGANIC & MEDICINAL CHEMISTRY LETTERS, Bd. 16, 2006, Seiten 5408-5413, XP002684625, GBELSEVIER SCIENCE LTD. ISSN: 0968-0896
- A. BEN-ASULY ET AL: "A thermally switchable latent ruthenium olefin metathesis catalyst", ORGANOMETALLICS., Bd. 27, 2008, Seiten 811-813, XP002684626, ACS, WASHINGTON, DC. ISSN: 0276-7333 in der Anmeldung erwähnt

## Beschreibung

Die vorliegende Anmeldung schließt durch Verweis die am 12. Juli 2011 eingereichte vorläufige US-Anmeldung 61/506,679 ein.

Die vorliegende Erfindung betrifft ein Verfahren zur Herstellung von Cyclohepten oder Derivaten davon durch katalytische Ringschlussmetathese von 1,8-Dienen der Formel I 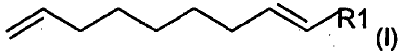 sowie deren Derivaten,
wobei R1 eine Alkylgruppe mit 1 bis 20 C-Atomen ist,
und wobei Derivate der 1,8-Diene der Formel t solche Verbindungen sind, die ausgehend von den 1,8-Dienen der Formel I an Position 2 bis 7 unabhängig voneinander mit einem oder mehreren Substituenten R2 modifiziert sind, wobei R2 ein Rest ist ausgewählt aus der Gruppe bestehend aus Alkylgruppe mit 1 bis 4 C-Atomen, Alkoxygruppe mit 1 bis 4 C-Atomen, Phenyl-Gruppe, Halogenid, Aminogruppe, Hydroxygruppe und Sulfonsäuregruppe,
wobei die Ringschlussmetathese in einem Reaktionsgemisch in Gegenwart eines inerten Lösemittels durchgeführt wird, und
wobei das entstehende Cyclohepten bzw. dessen Derivat während der Reaktion dem Reaktionsgemisch destillativ entfernt und isoliert wird und das 1,8-Dien der Formel I bzw. dessen Derivat dem Reaktionsgemisch so zudosiert wird, dass die Konzentration von 1,8-Dien der Formel I bzw. dessen Derivat im Reaktionsgemisch 1 mol/l nicht überschreitet.

Verbindungen, die einen Cycloheptan-Ring besitzen, werden sehr oft als Wirkstoffe genutzt. Marktreife haben zum Beispiel das Bencyclan (Fludilat®) als Kalziumkanalblocker, das Heptabarbital (Medomin®) als mittellang Wirkendes Barbiturat und die Incadronsäure (Bisphonal®) gegen maligne Hypercalcaemie.

Alle diese Wirkstoffe werden ausgehend von Cycloheptanon bzw. von Cycloheptylamin synthetisiert. Viele andere in der Literatur beschriebene Wirkstoffe werden ausgehend von anderen Cycloheptanderivaten synthetisiert. Von Bedeutung sind unter anderem Cycloheptancarbaldehyd sowie die daraus leicht herstellbare Cycloheptancarbonsäure und das Cycloheptancarbonsäurechlorid. Bisher ist aber lediglich Cycloheptanon (Suberon) technisch über einen relativ langen und aufwendigen Syntheseweg zugänglich.

EP 0 632 004 beschreibt die Synthese von Cycloheptanon durch Zyklisierung von Korksäuredinitril bei hoher Temperatur in Anwesenheit von Wasser an einem Si/Ti-Mischoxidkatalysator. Die Ausbeute ist zwar mit 85% relativ gut aber die Standzeit des Katalysators ist nur mäßig. Korksäuredinitril muss dabei in einer mehrstufigen Synthese hergestellt werden: Ausgehend von 1,6-Hexandiol, das mit Phosgen in 1,6-Dichlorhexan umgewandelt wird, und anschließend mit NaCN umgesetzt wird oder ausgehend von Korksäure über Korksäureamid und anschließender Wasserabspaltung mit P₄O₁₀. Beide Wege sind sehr lang und bieten nur einen Zugang zu Cycloheptanon.

Eine Synthese aller oben genannten Ausgangsbausteine (Cycloheptanon, Cycloheptylamin, Cycloheptancarbaldehyd, Cycloheptancarbonsäure und Cycloheptancarbonsäurechlorid) kann deutlich einfacher ausgehend von Cyclohepten als zentralem Baustein erfolgen:

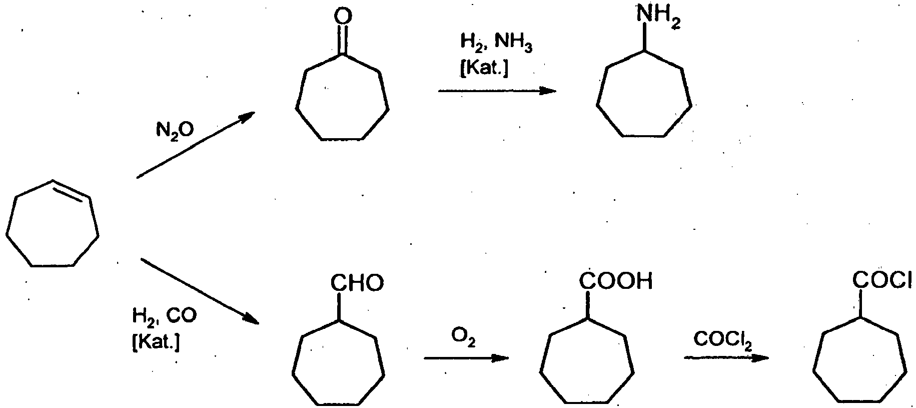

Cyclohepten kann durch Ringschlussmetathese (RCM) hergestellt werden ausgehend von dem α,ω- Olefin 1,8-Nonadien, das mit seinen zwei endständigen C-C-Doppelbindungen unter Abspaltung des flüchtigen Ethens zyklisiert (Ben-Asuly et al., Organometallics (2008), 27:811-813). Nachteilig sind dabei vor allem die fehlende technische Verfügbarkeit des Edukts sowie die relativ hohe Verdünnung. Die Herstellung von Cyclohepten oder dessen Derivaten durch Ringschlussmetathese ausgehend von unsymmetrischen α,ω- Olefinen oder gar von Dienen mit nur einer terminalen C-C-Doppelbindung wird in der Literatur nicht beschrieben. Vielmehr wird in der Literatur davon ausgegangen, dass bei Einsatz von unsymmetrischen Dienen die Dimerisierung durch acyclische Dien-Metathese (ADMET) bevorzugt ist (Conrad & Fogg, Curr Org Chemisty (2006), 10:185-202).

Zurzeit existiert kein einfacher technischer Zugang zu Cyclohepten. Ein solcher Zugang, ausgehend von technisch gut verfügbaren Ausgangsstoffen, wäre somit wünschenswert.

In COMMUNICATIONS, Bd. 46, 2010, Seiten 7145-7147, SEINSTITUTE OF INORGANIC AND PHYSICAL CHEMISTRY, STOCKHOLM, ISSN: 0366-5607 wird ein Verfahren zur Herstellung von Cyclohepten ausgehend von einem unsubstituierten 1,8-Dien erwähnt.

Als der Erfindung zugrunde liegende Aufgabe kann daher erachtet werden die Bereitstellung eines Verfahrens zur Herstellung von Cyclohepten, das von alternativen, insbesondere von technisch gut verfügbaren, Ausgangsstoffen ausgeht.

Entsprechend betrifft die vorliegende Erfindung ein Verfahren zur Herstellung von Cyclohepten oder Derivaten davon durch katalytische Ringschlussmetathese von 1,8-Dienen der Formel I 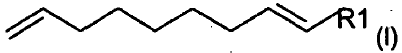 sowie deren Derivaten,
wobei R1 eine Alkylgruppe mit 1 bis 20 C-Atomen insbesondere mit 4, 6, 8 oder 10 C-atomen ist,
und wobei Derivate der 1,8-Diene der Formel I solche Verbindungen sind, die ausgehend von den 1,8-Dienen der Formel I an Position 2 bis 7 unabhängig voneinander mit einem oder mehreren Substituenten R2 modifiziert sind, wobei R2 ein Rest ist ausgewählt aus der Gruppe bestehend aus Alkylgruppe mit 1 bis 4 C-Atomen, Alkoxygruppe mit 1 bis 4 C-Atomen, Phenyl-Gruppe, Halogenid, Aminogruppe, Hydroxygruppe und Sulfonsäuregruppe,
wobei die Ringschlussmetathese in einem Reaktionsgemisch in Gegenwart eines inerten Lösemittels durchgeführt wird, und
wobei das entstehende Cyclohepten bzw. dessen Derivat während der Reaktion dem Reaktionsgemisch destillativ entfernt und isoliert wird und das 1,8-Dien der Formel I bzw. dessen Derivat dem Reaktionsgemisch so zudosiert wird, dass die Konzentration von 1,8-Dien der Formel I bzw. dessen Derivat im Reaktionsgemisch 1 mol/l nicht überschreitet.
Vorzugsweise weisen die 1,8-Diene der Formel I bzw. deren Derivate keine konjugierten Doppelbindungen auf. Vorzugsweise handelt es sich bei dem 1,8-Dien der Formel I um Heptadeca1,8-dien.

Alkylgruppen im Sinne der Erfindung sind aliphatische Kohlenwasserstoffreste ohne Heteroatome. Sie können verzweigt oder unverzeigt und gesättigt oder ungesättigt sein. Vorzugsweise sind sie gesättigt und unverzweigt.

Derivate der 1,8-Diene der Formel I sind solche Verbindungen, die ausgehend von den 1,8-Dienen der Formel I an Position 2 bis 7 unabhängig voneinander mit einem oder mehreren, vorzugsweise mit 1 bis 3 Substituenten R2 modifiziert sind. Dabei ist R2 eine Alkylgruppe mit 1 bis 4 C-Atomen, eine Alkoxygruppe mit 1 bis 4 C-Atomen, eine Phenyl-Gruppe, ein Halogenid, vorzugsweise Chlorid oder Bromid, eine Aminogruppe, eine Hydroxygruppe oder eine Sulfonsäuregruppe.

Grundsätzlich können für die Ringschlussmetathese von den 1,8-Dienen der Formel I bzw. deren Derivate alle denkbaren Metathesekatalysatoren verwendet werden. Da die Reaktion eine positive Aktivierungsentropie besitzt (aus einem Molekül werden zwei erzeugt), wird die Reaktion bei höheren Temperaturen begünstigt. Deshalb können für diese Reaktion auch klassische heterogene Katalysatoren für die Hochtemperatur-Gasphasenmetathese herangezogen werden, wie beispielsweise WO₃/SiO₂-Katalysatoren. Als heterogene Katalysatoren werden beispielsweise neben WO₃/SiO₂ auch Re₂O₇/Al₂O₃-Katalysatoren eingesetzt. Als homogene Metathesekatalysatoren werden üblicherweise Salze oder Komplexe des Wolframs oder des Rheniums (z. B. WCl₆ oder CH₃Re(CO)₅) oder Metall-Carben-Komplexe, die auf Ruthenium oder Molybdän basieren (Grubbs-Katalysator der ersten Generation, Grubbs-Katalysator der zweiten Generation, Schrock-Katalysator, Hoveyda-Grubbs-Katalysator), verwendet. Die Katalysatoren auf Re-, Mo- oder W-Basis werden häufig in Kombination mit einem Co-Katalysator (meist metallorganische Komplexe von Hauptgruppenelementen wie Aluminiumalkyle, Aluminiumalkylchloride oder Zinkalkyle) und einem Aktivator (beispielsweise Sauerstoffhaltige Verbindungen wie Ethanol oder Diethylether) eingesetzt. Geeignete Metathesekatalysatoren sind in der Literatur beschrieben (bspw. US 5,969,170, US 6,111,121, US 6,921,735, US11/094,102, US 6,759,537, EP 993465, US 6,635,768, WO 2007/003135, WO 2008/065187, EP1468004, DE102008008299). Geeignete Metathesekatalysatoren sind Ruthenium-Carben-Komplexe, wie beispielsweise Dichloro(3-methyl-2-butenyliden)bis(tricyclopentylphosphin)ruthenium(II), Isopentenyliden(1,3-dimesitylimidazolidin-2-yliden) (tricyclohexylphosphin)ruthenium(II)dichlorid, Benzyliden-bis(tricyclohexylphosphin)dichlororuthenium, 1,3-Bis (2,4,6-trimethylphenyl)-2(imidazolidinyliden)dichloro(phenylmethylen)(tricyclohexylphosphin)ruthenium, Dichloro(o-isopropoxyphenylmethylen)(tricyclohexylphosphin)ruthenium(II), (1,3-Bis-(2,4,6-trimethyl-phenyl)-2-imidazolidinyliden)dichloro(o-isopropoxyphenylmethylen)ruthenium, 1,3-Bis(2-methylphenyl)-2-imidazolidinyliden]dichloro(phenylmethylen)(tricyclohexylphosphin)-ruthenium(II), Dichloro[1,3-bis(2-methylphenyl)-2-imidazolidinyliden](2-isopropoxyphenyl-methylen)ruthenium(II), Dichloro[1,3-bis(2,4,6-trimethylphenyl)-2-imidazolidinyliden][3-(2-pyridinyl)propyliden]ruthenium(II), Dichloro[1,3-bis(2,4,6-trimethylphenyl)-2-imidazolidinyliden]-(benzyliden)bis(3-bromopyridin)ruthenium(II), [1,3-Bis(2,6-di-i-propylphenyl)-4,5-dihydroimidazol-2-yliden]-[2-i-propoxy-5-(trifluoroacetamido)phenyl]methylenruthenium(II)dichlorid, Bis-(tricyclohexylphosphin)-3-phenyl-1H-inden-1-yliden-ruthenium(II)dichlorid, Bis(tricyclo-hexylphosphin)[(phenylthio)methylen]ruthenium(II)dichlorid, Bis(tricyclohexylphosphin)-[(phenylthio)methylen]ruthenium(II)dichlorid, 1,3-Bis(2,4,6-trimethylphenyl)-4,5-dihydroimidazol-2-yliden[2-(i-propoxy)-5-(N,N-dimethylaminosulfonyl)phenyl]methylen ruthenium(II)dichlorid, 1,3-Bis(2,4,6-trimethylphenyl)-4,5-dihydroimidazol-2-yliden[2-(i-propoxy)-5-(N,N-dimethyl-aminosulfonyl)phenyl]methylen-ruthenium(II)dichlorid, [1,3-Bis(2,4,6-trimethylphenyl)-2-imidazolidinyliden]-[2-[[(4-methylphenyl)imino]methyl]-4-nitrophenolyl]-[3-phenyl-1H-inden-1-yliden]ruthenium(II)chlorid; [1,3-Bis(2,4,6-trimethylphenyl)-2-imidazolidinyliden]-[2-[[(2-methylphenyl)imino]methyl]phenolyl]-[3-phenyl-1H-inden-1-yliden]ruthenium(II)chlorid, 3-Phenyl-1H-inden-1-yliden[bis(i-butylphoban)]ruthenium(II)dichlorid, {[2-(i-propoxy)-5-(N,N-dimethylaminosulfonyl)phenyl]methylen}(tricyclohexylphosphin)ruthenium(II)dichlorid, Tricyclo-hexylphosphin [1,3-bis(2,4,6-trimethylphenyl)-4,5-dihydroimidazol-2-yliden][(phenylthio)-methylen]ruthenium(II)dichlorid, Tricyclohexylphosphin[1,3-bis(2,4,6-trimethylphenyl)imidazol-2-yliden][3-phenyl-1H-inden-1-yliden]ruthenium(II)dichlorid, Tricyclohexylphosphin[1,3-bis(2,4,6-trimethylphenyl)imidazol-2-yliden][2-thienylmethylen]ruthenium(II)dichlorid, Tricyclohexyl-phosphin[2,4-dihydro-2,4,5-triphenyl-3H-1,2,4-triazol-3-yliden][2-thienylmethylen]ruthenium(II)-dichlorid, Tricyclohexylphosphin[4,5-dimethyl-1,3-bis(2,4,6-trimethylphenyl)imidazol-2-yliden][2-thienylmethylen]ruthenium(II)dichlorid, Tricyclohexylphosphin[3-phenyl-1H-inden-1-yliden][1,3-bis(2,4,6-trimethylphenyl)-4,5-dihydroimidazol-2-yliden]ruthenium(II)dichlorid, Trifluoroacetato-[4,5-dihydro-1,3-bis(2,4,6-trimethylphenyl)imidazol-2-yliden]tetra(2,2-dimethylpropannitril)-ruthenium(II)trifluoroacetate oder Tri(i-propoxy)phosphin(3-phenyl-1H-inden-1-yliden)[1,3-bis-(2,4,6-trimethylphenyl)-4,5-dihydroimidazol-2-yliden]ruthenium(II)dichlorid.

Abhängig von dem eingesetzten Katalysatortyp wird die Reaktion bei milden Temperaturen (beispielsweise 0 bis 100 °C) oder bei erhöhter Temperatur (beispielsweise 100 bis 500 °C) durchgeführt.

Die katalytische Ringschlussmetathese kann in Flüssigphase oder in Gasphase erfolgen. Sie kann sowohl als homogene Katalyse wie auch als heterogene Katalyse durchgeführt werden.

Bevorzugt werden für die erfindungsgemäße Ringschlussmetathese Ru-Katalysatoren eingesetzt, beispielsweise die Ru-Katalysatoren der Formeln II (Mes= Mesityl, Ph=Phenyl, Pr= Propyl), III (mit Cy= Cyclohexyl, Ph= Phenyl) oder IV (Hoveyda-Grubbs Katalysator der 2ten Generation, mit Mes= Mesityl)

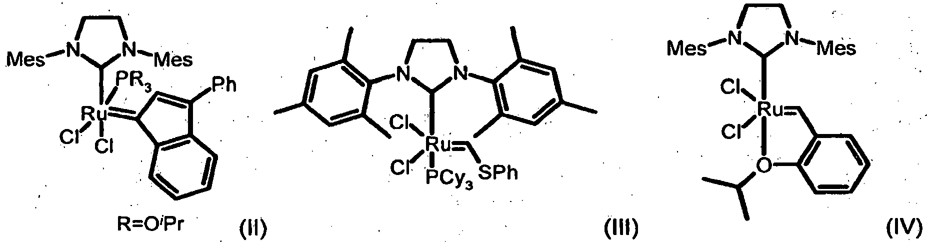

Bei der Ringschlussmetathese von 1,8-Dienen der Formel I bzw. dessen Derivat wird Cyclohepten bzw. dessen Derivat unter Abspaltung eines Olefins der Formel V 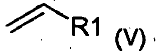 als Nebenprodukt gebildet. Dieses Nebenprodukt kann unter den Reaktionsbedingungen unter Abspaltung von Ethen dimerisieren zu dem Olefin der Formel VI

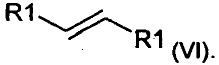

Im Falle der Ringschlussmetathese von Heptadeca-1,8-dien ist das abgespaltene Nebenprodukt der Formel V 1-Decen und das entsprechende Dimerisierungsprodukt der Formel VI ist 9-Octadecen. Die Olefine der Formeln V und VI sowie insbesondere das 1-Decen und das 9-Octadecen sind ebenfalls technisch bedeutsame Wertprodukte, die aus dem Reaktionsgemisch isoliert werden können. Die Olefine der Formel VI, bspw. das 9-Octadecen, fallen üblicherweise als Sumpfprodukt bei der erfindungsgemäßen Ringschussmetathese-Reaktion an. Die Olefine der Formeln V und VI können in einer Koppelproduktion neben dem Cyclohepten, bzw. dessen Derivat bei dem erfindungsgemäßen Verfahren gewonnen werden.

Die erfindungsgemäße Ringschlussmetathese wird in kontinuierlicher Fahrweise durchgeführt. Die erfindungsgemäße Ringschlussmetathese wird als Reaktivdestillation durchgeführt.

Bei einer Durchführung der erfindungsgemäßen Ringschlussmetathese als kontinuierliche Reaktivdestillation wird das Edukt (1,8-Dien der Formel I oder ein Derivat davon), in einem inerten Lösemittel, kontinuierlich der Reaktivzone einer Rektifikationskolonne mit Auf- und Abtriebsteil zugeführt. In der Reaktivzone ist der Metathese-Katalysator vorgelegt. Die Reaktion erfolgt vorzugsweise bei einer Temperatur von 0 bis 500 °C, besonders bevorzugt von 20 bis 100 °C und unter einem Druck von vorzugsweise 1 bis 200 mbar, besonders bevorzugt von 5 bis 150 mbar. Die Produkte (Cyclohepten bzw. dessen Derivate sowie die bei der Metathese abgespaltenen Olefine bzw. deren Dimere) werden in der Rektifikationskolonne destillativ aus dem Reaktionsgemisch abgetrennt und kontinuierlich abgezogen. Vorzugsweise wird ein Lösemittel mit einem Siedepunkt gewählt, der einen weitestgehenden Verbleib des Lösemittels in des Reaktivzone der Kolonne ermöglicht. Mit den Produkten aus der Kolonne abgezogenes Lösemittel wird vorzugsweise von den Produkten abgetrennt (bspw. destillativ) und zusammen mit dem Edukt der Reaktion wieder zugeführt. Die Reaktionsbedingungen werden so eingestellt, dass die Konzentration des Edukts in der Reaktivzone einen Wert von maximal 1 mol/l, bevorzugt maximal 0,5 mol/l nicht überschreitet und beispielsweise in einem Konzentrationsbereich von 0,005 bis 0,5 mol/l, bevorzugt von 0,01 bis 0,1 mol/l liegt. Geeignete Lösemittel sind beispielsweise hochsiedende aromatische Kohlenwasserstoffe wie Xylol, Toluol oder Cymol, halogenierte Kohlenwasserstoffe wie Dichlormethan oder Dichlorethan, halogenierte Aromaten wie Dichlorbenzole und Diphenylether. Die eingesetzte Menge des Metathese-Katalysators beträgt vorzugsweise maximal 0,1 mol-%, besonders bevorzugt maximal 0,05 mol-% bezogen auf das Edukt. Vorzugsweise wird dem Reaktionsgemisch ein Schleppmittel zugesetzt, das die destillative Abtrennung des Produkts während der Reaktion verbessert. Bevorzugt wird das Schleppmittel nach der destillativen Abtrennung vom Produkt getrennt und dem Reaktionsgemisch wieder zugeführt.

Das 1,8-Dienen der Formel I für die erfindungsgemäße katalytische Ringschlussmetathese kann hergestellt werden durch katalytische Decarbonylierung (Trost & Fleming (Hrsg.), Comprehensive Organic Synthesis. Ist. Ed., Elsevier (1991) 3:1040-1042) oder Decarboxylierung (Carey & Sundberg, Advanced Organic Chemistry, Plenum Press (1990), 3rd. Ed., New York, S. 649-651) einer Carbonsäure, bzw. eines Carbonsäurederivats der Formel VII

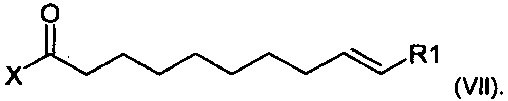

X ist ein Halogenid, bevorzugt Chlorid, ein H-Atom, eine OH-Gruppe oder eine andere geeignete Abgangsgruppe wie beispielsweise eine Acylgruppe, vorzugsweise Acetyl, Pivaloyl oder Benzoyl. Grundsätzlich sind auch andere Carbonsäureaktivierungen wie Imidazolide oder Aktivester möglich. Derivate der 1,8-Diene der Formel I können in analoger Weise hergestellt werden, ausgehend von den entsprechenden Derivaten der Carbonsäure bzw. Carbonsäurederivaten der Formel VII. Heptadeca-1,8-dien kann beispielsweise durch katalytische Decarbonylierung oder oxidative Decarboxylierung von Ölsäure oder eines Ölsäurederivats, wie das Ölsäurechlorid, hergestellt werden. In entsprechender Weise kann ein für die Ringschlussmetathese zum Cyclohepten geeignetes 1,8-Dien der Formel I z. B. auch durch katalytische Decarbonylierung oder oxidative Decarboxylierung von Myristoleinsäure, Palmitoleinsäure, Elaidinsäure oder Gadoleinsäure bzw. deren Säurederivate hergestellt werden. Die entsprechende Verwendung von mehrfach ungesättigten Carbonsäuren bzw. Carbonsäurederivaten wie bspw. Linolsäure ist auch möglich, kann aber prinzipiell bei der Ringschlussmetathese zu ungesättigten Cycloaliphaten mit größeren Ringgrößen als Nebenprodukt führen.

Solche katalytischen Decarbonylierungen bzw. oxidativen Decarboxylierungen sind prinzipiell in der Literatur bereits beschrieben (Blum et al., J Am Chem Soc (1967) 89:2338-2341; Ohno & Tsuji, J Am Chem Soc (1968) 90:99-107; Tsuji & Ohno, Synthesis (1969) 157-169). Goossen & Rodríguez (Chem Comm (2004) 724-725) beschreiben beispielsweise die Umsetzung von Ölsäure zum Heptadeca-1,8-dien in Anwesenheit eines Überschusses von Pivalsäureanhydrid und eines Pd-Katalysators mit einer Ausbeute von 69%. Ebenfalls bekannt ist die Decarbonylierung von Ölsäure in Anwesenheit eines Überschusses an Essigsäureanhydrid und PdCl₂(PPh₃)₂ als Katalysator (Miller et al., J Org Chem (1993) 58:18-20). Die gleiche Reaktion mit einem ähnlichen Katalysatorsystem wurde bereits in US 5,077,447 und in US 3,109,040 beschrieben.

Carbonsäuren der Formel VII mit X = OH, wie beispielsweise Ölsäure, werden bei der katalytischen Decarbonylierung bzw. oxidative Decarboxylierung unter Abspaltung von CO und Wasser zu 1,8-Dienen der Formel I, wie beispielsweise Heptadeca-1,8-dien umgesetzt. Da Wasser bei dieser Umsetzung eine schlechte Abgangsgruppe ist, sind die Carbonsäuren der Formel VII mit X = OH keine besonders guten Substrate für diese Umsetzung. Vorteilhafterweise wird die Carbonsäure der Formel VII mit X = OH in situ aktiviert, beispielsweise durch Bildung eines gemischten Anhydrides mit Essigsäure- oder Pivalsäureanhydrid, oder es wird ein Carbonsäurederivat mit einer besseren Abgangsgruppe eingesetzt, wie z. B. ein Säurechlorid. Die Reaktion kann dann als eine Retro-Koch-Carbonylierung angesehen werden. Anstelle der Säure kann auch der Aldehyd eingesetzt werden, wobei dann CO und H₂ abgespalten werden und die Reaktion als eine Retro-Hydroformylierung angesehen werden kann. Es können reine Carbonsäuren bzw. Carbonsäurederivate der Formel VII eingesetzt werden oder auch Gemische, die diese enthalten. Solche Gemische enthalten die Carbonsäuren bzw. Carbonsäurederivate der Formel VII vorzugsweise zu einem Anteil von mindestens 50 Gew.-%.

Da Ölsäure bzw. Ölsäurechlorid technisch verfügbar sind, ist deren Einsatz für die Herstellung von Heptadeca-1,8-dien besonders bevorzugt.

Für die katalytische Decarbonylierung oder oxidative Decarboxylierung von Carbonsäuren, bzw. Carbonsäurederivate der Formel VII können alle in der Literatur beschriebenen Katalysatoren können eingesetzt werden. Besonders bevorzugt werden Pd(II)- und Rh(I)-Komplexe als Katalysatoren eingesetzt.

Die katalytische Decarbonylierung oder oxidative Decarboxylierung von Carbonsäuren, bzw. Carbonsäurederivate der Formel VII zu 1,8-Dienen der Formel I erfolgt bevorzugt als Reäktivdestillation in Semi-Batch Fahrweise, wobei die Carbonsäure bzw. das Carbonsäurederivat der Formel VII gleichmäßig zum Reaktor zudosiert wird und die leicht flüchtigen Produkte möglichst rasch entfernt werden. Um dies zu bewerkstelligen wird die Reaktionstemperatur abhängig vom Katalysator so gewählt, dass eine geeignete Reaktionsgeschwindigkeit erhalten wird. Passend zu dieser Reaktionstemperatur wird dann der Druck so gewählt, dass bei diesem Druck der Siedepunkt des 1,8-Diens der Formel I mindestens 10 °C unter der Reaktionstemperatur liegt. Die Reaktion kann sowohl in Substanz als auch in Anwesenheit eines hochsiedenden, unter den Reaktionsbedingungen inerten Lösemittels durchgeführt werden. Unter einem "hochsiedenden Lösemittel" ist in diesem Zusammenhang ein Lösemittel zu verstehen, dessen Siedepunkt unter den gegebenen Reaktionsbedingungen höher ist als die gewählte Reaktionstemperatur.

In einer bevorzugten Variante der katalytischen Decarbonylierung oder oxidativen Decarboxylierung wird der Katalysator zunächst in einem hochsiedenden Lösemittel oder einer ionischen Flüssigkeit gelöst und diese Lösung auf einen porösen, inerten Träger aufgebracht. Der Katalysator wird dann in einem Festbettreaktor eingesetzt.

Vor dem Einsatz in der erfindungsgemäßen Ringschlussmetathese wird das 1,8-Dien der Formel I vorzugsweise gereinigt. Die Reinigung kann auch mehrstufig sein. Beispielsweise kann man von Ölsäurechlorid ausgehend den gasförmigen Reaktionsaustrag zuerst kondensieren, anschließend das gebildete wässrige HCl durch Phasentrennung abtrennen, dann die organische Phase mit Wasser und/oder einer verdünnten Lauge gegebenenfalls auch mehrfach waschen, und schließlich die organische Phase durch Destillation reinigen.

Das erfindungsgemäß hergestellte Cyclohepten kann in einem weiteren Schritt beispielsweise mittels N₂O zum Cycloheptanon oxidiert werden. Möglich ist auch eine zweistufige Sequenz aus Epoxidierung und Umlagerung des Epoxids zum Suberon. Das Cycloheptanon wiederum kann in einem anschließenden Schritt beispielsweise mittels Wasserstoff und Ammoniak katalytisch zum Cycloheptylamin umgesetzt werden (reduktive Aminierung). Alternativ kann das erfindungsgemäß hergestellte Cyclohepten auch beispielsweise mittels Wasserstoff und Kohlenmonoxid katalytisch zum Cycloheptancarbaldehyd umgesetzt werden (Hydroformylierung). Dieses kann in einem anschließenden Schritt beispielsweise unter Sauerstoffzugabe zur Cycloheptancarbonsäure oxidiert werden. Cycloheptancarbonsäure kann auch direkt aus Cyclohepten, bspw. gemäß WO 01/05738 durch Umsetzung mit Ameisensäure, hergestellt werden. Die Cyc-Ioheptancarbonsäure wiederum kann beispielsweise mittels Phosgen zu Cycloheptancarbonsäurechlorid umgesetzt werden. In analoger Weise können auch die entsprechenden Derivate des Cycloheptanons, Cycloheptylamins, Cycloheptancarbaldehyds, der Cycloheptancarbonsäure oder des Cycloheptancarbonsäurechlorids hergestellt werden, ausgehend von dem entsprechenden Derivat des Cycloheptens mit einem oder mehreren Substituenten R2 am Cycloheptenring, wobei dieses erfindungsgemäß aus solchen Verbindungen hervorgeht, die ausgehend von den 1,8-Dienen der Formel I an Position 2 bis 7 unabhängig voneinander mit einem oder mehreren, bevorzugt 1 bis 3 Substituenten R2 modifiziert sind. Dabei ist R2 eine Alkylgruppe mit 1 bis 4 C-Atomen, eine Alkoxygruppe mit 1 bis 4 C-Atomen, eine Phenyl-Gruppe, ein Halogenid, vorzugsweise Chlorid oder Bromid, eine Aminogruppe, eine Hydroxygruppe oder eine Sulfonsäuregruppe.

Die Erfindung wird nun durch die nachfolgenden, nichtlimitierenden Beispiele näher erläutert.

### Beispiel 1

Eine Lösung des Ru-Komplexes der Formel II (73 mg, 80 µmol) als Metathese-Katalysator in Diephenylether (222 g) wurde unter Vakuum (8 mbar) auf 85 °C erwärmt. Zu dieser Mischung wurde eine Lösung von 1,8-Heptadecadien (21,9 g, 90 Gew.-% Reinheit, 83 mmol) in Toluol (22 ml) mittels einer Spritzenpumpe über einen Zeitraum von 4 h gleichmäßig eingetropft, während das Produkt Cyclohepten und andere Leichtsieder (bspw. 1-Decen und Toluol) ständig in eine Vorlage abdestilliert wurden. Am Ende der Reaktion war eine vollständige Umsetzung des Edukts festzustellen. Die Menge an Cyclohepten in dem Destillat wurde mittels GC-Ahalyse bestimmt und entsprach 4,80 g (50 mmol, 60 % der theoretischen Ausbeute).

### Beispiel 2

Die Reaktion wurde analog zu Beispiel 1 durchgeführt, jedoch unter Einsatz von 74,3 mg (83 µmol) des Ru-Komplexes der Formel III als Metathese-Katalysator. Auch in diesem Fall wurde das Edukt vollständig umgesetzt. Das Destillat enthielt 4,97 g (52 mmol) Cyclohepten, entsprechend 63 % der theoretischen Ausbeute.

### Beispiel 3

Die Reaktion wurde analog zu Beispiel 1 durchgeführt, jedoch unter Einsatz von nur 36,6 mg, 42 µmol) des Ru-Komplexes der Formel II. Auch in dem Fall war am Ende der Reaktion die vollständige Umsetzung des Edukts festzustellen. Das Destillat enthielt 4,23 g (44 mmol) Cyclohepten, entsprechend 53 % der theoretischen Ausbeute.

### Beispiel 4

Die Reaktion wurde analog zu Beispiel 1 durchgeführt, jedoch unter Einsatz von 47,7 mg (76 µmol) des Ru-Komplexes der Formel IV als Metathese-Katalysator. Auch in diesem Fall wurde das Edukt vollständig umgesetzt. Das Destillat enthielt 5,57 g (58 mmol) Cyclohepten, entsprechend 70 % der theoretischen Ausbeute.

### Beispiel 5

Zu einer Lösung von 1,8-Heptadecadien (23,3 g, 90 Gew.-% Reinheit, 89 mmol) in Diphenylether (236 g) wurden bei Raumtemperatur 50,6 mg (81 µmol) des Metathese-Katalysators der Formel IV hinzugefügt. Die Reaktionsmischung wurde unter Vakuum (8 mbar) auf 85 °C erwärmt. Das gebildete Produkt Cyclohepten und andere Leichtsieder (bspw. 1-Decen) wurden während der Reaktion gleichmäßig in eine Vorlage destilliert. Nach einer Reaktionszeit von 24 h wurde mittels GC-Analyse ein Umsatz des Edukts von >95 % festgestellt. Das Destillat enthielt laut GC-Analyse eine Menge von 1,78 g (19 mmol) Cyclohepten, entsprechend 20 % der theoretischen Ausbeute. Nach Zugabe einer weiteren Portion des Metathese-Katalysators (50 mg, 80 µmol) wurde die Reaktion für weitere 6 h fortgesetzt. Nur Spuren von zusätzlichem Cyclohepten konnten daraufhin in der Vorlage nachgewiesen werden.

### Beispiel 6

Zu einer Lösung von 1,8-Heptadecadien (2,12 g, 90 Gew.-% Reinheit, 8 mmol) in Diphenylether (21 g) wurden bei 85 °C 2,56 mg (4 µmol, aus Maßlösung) des Metathese-Katalysators der Formel IV hinzugefügt. Nach einer Reaktionszeit von 3 h wurde das Reaktionsgemisch analysiert. Das Edukt war zu 88 % umgesetzt und das Reaktionsgemisch enthielt 110 mg (1 mmol) Cyclohepten, entsprechend 15 % der theoretischen Ausbeute.

## Patentansprüche

1. Verfahren zur Herstellung von Cyclohepten oder Derivaten davon durch katalytische Ringschlussmetathese von 1,8-Dienen der Formel I 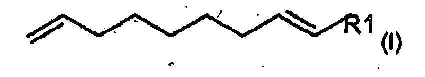 sowie deren Derivaten,
wobei R1 eine Alkylgruppe mit 1 bis 20 C-Atomen ist,
und wobei Derivate der 1,8-Diene der Formel I solche Verbindungen sind, die ausgehend von den 1,8-Dienen der Formel I an Position 2 bis 7 unabhängig voneinander mit einem oder mehreren Substituenten R2 modifiziert sind, wobei R2 ein Rest ist ausgewählt aus der Gruppe bestehend aus Alkylgruppe mit 1 bis 4 C-Atomen, Alkoxygruppe mit 1 bis 4 C-Atomen, Phenyl-Gruppe, Halogenid, Aminogruppe, Hydroxylgruppe und Sulfonsäuregruppe,
wobei die Ringschlussmetathese in einem Reaktionsgemisch in Gegenwart eines inerten Lösemittels durchgeführt wird, und
wobei das entstehende Cyclohepten bzw. dessen Derivat während der Reaktion dem Reaktionsgemisch destillativ entfernt und isoliert wird und das 1,8-Dien der Formel I bzw. dessen Derivat dem Reaktionsgemisch so zudösiert wird, dass die konzentration von 1,8-Dien der Formel I bzw. dessen Derivat im Reaktionsgemisch 1 mol/l nicht überschreitet.

2. Das Verfahren gemäß Anspruch 1, wobei das Reaktionsgemisch zusätzlich ein Schleppmittel enthält.

3. Das Verfahren gemäß Anspruch 1 oder 2, wobei ein Ruthenium-Carben-Komplex als Katalysator für die Ringschlussmetathese eingesetzt wird.

4. Das Verfahren gemäß einem der. Ansprüche 1 bis 3, wobei das bei der Ringschlussmetathese des 1,8-Diens der Formel I bzw. dessen Derivats neben dem Cyclohepten bzw. dessen Derivat entstehende Olefin der Formel V 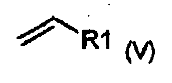 und/oder dessen Dimensierungsprodukt, das Olefin der Formel VI 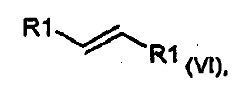 als Koppelprodukt des Verfahrens ebenfalls isoliert wird.

5. Das Verfahren gemäß einem der Ansprüche 1 bis 4, wobei das 1,8-Dien der Formel I bzw. dessen Derivat hergestellt wird durch katalytische Dezarbonylierung oder oxidative Decarboxylierung einer Carbonsäure oder eines Carbonsäurederivats der Formel VII 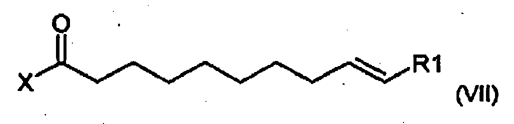 bzw. dessen Derivat, wobei X eine Abgangsgruppe ist.

6. Das Verfahren gemäß Anspruch 5, wobei die Abgangsgruppe X ausgewählt ist aus der Gruppe bestehend aus H-Atom, Halogenid, OH-Gruppe und Acylgruppe.

7. Das Verfahren gemäß einem der Ansprüche 1 bis 6, wobei das 1,8-Dien der Formel I bzw. dessen Derivat vor dem Einsatz in der Ringschlussmetathese-Reaktion gereinigt wird.

8. Das Verfahren gemäß einem der Ansprüche 1 bis 7, wobei das 1,8-Dien der Formel I Heptadeca-1,8-dien ist, und wobei im Falle der Ansprüche 5 bis 7 die Carbonsäure oder das Carbonsäurederivat der Formel VII Ölsäure oder ein Ölsäurederivat ist.

9. Verfahren zur Herstellung von Cycloheptanon oder dessen Derivat durch Oxidation von Cyclohepten bzw. dessen Derivat, weiches hergestellt ist nach einem der Ansprüche 1 bis 8, wobei das Derivat die entsprechende Verbindung ist, die mit einem oder mehreren Resten R2 am Cycloheptenring substituiert ist, und wobei R2 ein Rest ist ausgewählt aus der Gruppe bestehend aus Alkylgruppe mit 1 bis 4 C-Atomen, AlKoxygruppe mit 1 bis 4 C-Atomen, Phenyl-Gruppe, Halogenid, Aminogruppe, Hydroxygruppe und Sulfonsäuregruppe.

10. Verfahren zur Herstellung von Cycloheptylamin oder dessen Derivat durch Umsetzung von Cycloheptanon bzw. dessen Derivat, hergestellt nach Anspruch 9, wobei das Derivat die entsprechende Verbindung ist, die mit einem oder mehreren Resten R2 am Cycloheptenring substituiert ist, und wobei R2 ein Rest ist ausgewählt aus der Gruppe bestehend aus Alkylgruppe mit 1 bis 4 C-Atomen, Alkoxygruppe mit 1 bis 4 C-Atomen, Phenyl-Gruppe, Halogenid, Aminogruppe, Hydroxygruppe und Sulfonsäuregruppe.

11. Verfahren zur Herstellung von Cycloheptancarbaldehyd oder dessen Derivat durch Umsetzung von Cyclohepten bzw. dessen Derivat, das hergestellt ist nach einem der Ansprüche 1 bis 8, wobei das Derivat die entsprechende Verbindung ist, die mit einem oder mehreren Resten R2 am Cycloheptenring substituiert ist, und wobei R2 ein Rest ist ausgewählt aus der Gruppe bestehend aus Alkylgruppe mit 1 bis 4 C-Atomen, Alkoxygruppe mit 1 bis 4 C-Atomen, Phenyl-Gruppe, Halogenid, Aminogruppe, Hydroxygruppe und Sulfonsäuregruppe.

12. Verfahren zur Herstellung von Cycloheptancarbonsäure oder dessen Derivat durch Umsetzung von Cyclohepten bzw. dessen Derivat, das hergestellt ist nach einem der Ansprüche 1 bis 8, wobei das Derivat die entsprechende Verbindung ist, die mit einem oder mehreren Resten R2 am Cycloheptenring substituiert ist, und wobei R2 ein Rest ist ausgewählt aus der Gruppe bestehend aus Alkylgruppe mit 1 bis 4 C-Atomen, Alkoxygruppe mit 1 bis 4 C-Atomen, Phenyl-Gruppe, Halogenid, Aminogruppe, Hydroxygruppe und Sulfonsäuregruppe.

13. Verfahren zur Herstellung von Cycloheptancarbonsäure oder deren Derivat durch Oxidation von Cycloheptancarbaldehyd bzw. dessen Derivat, hergestellt nach Anspruch 11, wobei das Derivat die entsprechende Verbindung ist, die mit einem oder mehreren Resten R2 am Cycloheptenring substituiert ist, und wobei R2 ein Rest ist ausgewählt aus der Gruppe bestehend aus Alkylgruppe mit 1 bis 4 C-Atomen, Alkoxygruppe mit 1 bis 4 C-Atomen, Phenyl-Gruppe, Halogenid, Aminogruppe, Hydroxygruppe und Sulfonsäuregruppe.

14. Verfahren zur Herstellung von Cycloheptancarbonsäurechlorid oder dessen Derivat durch Umsetzung von Cycloheptancarbonsäure, bzw. dessen Derivat, hergestellt nach Anspruch 12 oder 13 wobei das Derivat die entsprechende Verbindung ist, die mit einem oder mehreren Resten R2 am Cycloheptenring substituiert ist, und wobei R2 ein Rest ist ausgewählt aus der Gruppe bestehend aus Alkylgruppe mit 1 bis 4 C-Atomen, Alkoxygruppe mit 1 bis 4 C-Atomen, Phenyl-Gruppe, Halogenid, Aminogruppe, Hydroxygruppe und Sulfonsäuregruppe.

## Claims

1. A process for preparing cycloheptene or derivatives thereof by catalytic ring-closing metathesis of 1,8-dienes of the formula I 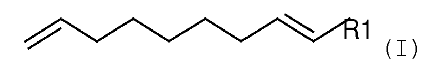 and derivatives thereof,
where R1 is an alkyl group having 1 to 20 carbon atoms,
and where derivatives of the 1,8-dienes of the formula I are those compounds which have been modified independently by one or more R2 substituents at positions 2 to 7 proceeding from the 1,8-dienes of the formula I, where R2 is a radical selected from the group consisting of alkyl group having 1 to 4 carbon atoms, alkoxy group having 1 to 4 carbon atoms, phenyl group, halide, amino group, hydroxyl group and sulfo group,
wherein the ring-closing metathesis is performed in a reaction mixture in the presence of an inert solvent, and
wherein the cycloheptene or derivative thereof formed is removed by distillation from the reaction mixture and isolated during the reaction, and the 1,8-diene of the formula I or derivative thereof is metered into the reaction mixture such that the concentration of 1,8-diene of the formula I or derivative thereof in the reaction mixture does not exceed 1 mol/l.

2. The process according to claim 1, wherein the reaction mixture additionally comprises an entraining agent.

3. The process according to claim 1 or 2, wherein a ruthenium-carbene complex is used as a catalyst for the ring-closing metathesis.

4. The process according to any of claims 1 to 3, wherein the olefin of the formula V formed in addition to the cycloheptene or derivative thereof in the ring-closing metathesis of the 1,8-diene of the formula I or derivative thereof 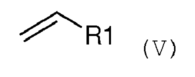 and/or the dimerization product thereof, the olefin of the formula VI 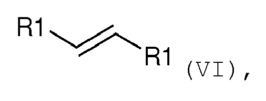 is likewise isolated as a coproduct of the process.

5. The process according to any of claims 1 to 4, wherein the 1,8-diene of the formula I or derivative thereof is prepared by catalytic decarbonylation or oxidative decarboxylation of a carboxylic acid or of a carboxylic acid derivative of the formula VII 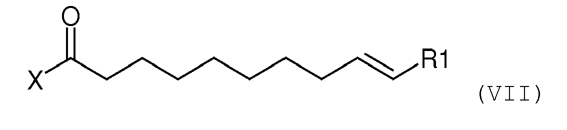 or derivative thereof, where X is a leaving group.

6. The process according to claim 5, wherein the leaving group X is selected from the group consisting of hydrogen atom, halide, OH group and acyl group.

7. The process according to any of claims 1 to 6, wherein the 1,8-diene of the formula I or derivative thereof is purified before use in the ring-closing metathesis reaction.

8. The process according to any of claims 1 to 7, wherein the 1,8-diene of the formula I is heptadeca-1,8-diene, and wherein, in the case of claims 5 to 7, the carboxylic acid or carboxylic acid derivative of the formula VII is oleic acid or an oleic acid derivative.

9. A process for preparing cycloheptanone or derivative thereof by oxidizing cycloheptene or derivative thereof prepared according to any of claims 1 to 8, where the derivative is the corresponding compound substituted by one or more R2 radicals on the cycloheptene ring, and where R2 is a radical selected from the group consisting of alkyl group having 1 to 4 carbon atoms, alkoxy group having 1 to 4 carbon atoms, phenyl group, halide, amino group, hydroxyl group and sulfo group.

10. A process for preparing cycloheptylamine or derivative thereof by converting cycloheptanone or derivative thereof prepared according to claim 9, where the derivative is the corresponding compound substituted by one or more R2 radicals on the cycloheptene ring, and where R2 is a radical selected from the group consisting of alkyl group having 1 to 4 carbon atoms, alkoxy group having 1 to 4 carbon atoms, phenyl group, halide, amino group, hydroxyl group and sulfo group.

11. A process for preparing cycloheptanecarbaldehyde or derivative thereof by converting cycloheptene or derivative thereof prepared according to any of claims 1 to 8, where the derivative is the corresponding compound substituted by one or more R2 radicals on the cycloheptene ring, and where R2 is a radical selected from the group consisting of alkyl group having 1 to 4 carbon atoms, alkoxy group having 1 to 4 carbon atoms, phenyl group, halide, amino group, hydroxyl group and sulfo group.

12. A process for preparing cycloheptanecarboxylic acid or derivative thereof by converting cycloheptene or derivative thereof prepared according to any of claims 1 to 8, where the derivative is the corresponding compound substituted by one or more R2 radicals on the cycloheptene ring, and where R2 is a radical selected from the group consisting of alkyl group having 1 to 4 carbon atoms, alkoxy group having 1 to 4 carbon atoms, phenyl group, halide, amino group, hydroxyl group and sulfo group.

13. A process for preparing cycloheptanecarboxylic acid or derivative thereof by oxidizing cycloheptanecarbaldehyde or derivative thereof prepared according to claim 11, where the derivative is the corresponding compound substituted by one or more R2 radicals on the cycloheptene ring, and where R2 is a radical selected from the group consisting of alkyl group having 1 to 4 carbon atoms, alkoxy group having 1 to 4 carbon atoms, phenyl group, halide, amino group, hydroxyl group and sulfo group.

14. A process for preparing cycloheptanecarbonyl chloride or derivative thereof by converting cycloheptanecarboxylic acid or derivative thereof prepared according to claim 12 or 13, where the derivative is the corresponding compound substituted by one or more R2 radicals on the cycloheptene ring, and where R2 is a radical selected from the group consisting of alkyl group having 1 to 4 carbon atoms, alkoxy group having 1 to 4 carbon atoms, phenyl group, halide, amino group, hydroxyl group and sulfo group.

## Revendications

1. Procédé pour la production de cycloheptène ou de dérivés de celui-ci, par métathèse cyclisante catalytique de 1,8-diènes de formule I 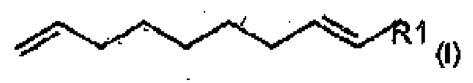 ainsi que de leurs dérivés,
R1 étant un groupe alkyle ayant de 1 à 20 atomes de carbone,
et les dérivés des 1,8-diènes de formule I étant les composés qui sont modifiés à partir des 1,8-diènes de formule I en position 2 à 7, indépendamment les uns des autres, par un ou plusieurs substituants R2, R2 étant un radical choisi dans le groupe constitué par un groupe alkyle ayant de 1 à 4 atomes de carbone, un groupe alcoxy ayant de 1 à 4 atomes de carbone, le groupe phényle, un halogénure, le groupe amino, le groupe hydroxy et le groupe sulfo,
la métathèse cyclisante étant effectuée dans un mélange réactionnel en présence d'un solvant inerte, et
le cycloheptène ou son dérivé, résultant, étant séparé par distillation du mélange réactionnel pendant la réaction et isolé et le 1,8-diène de formule I ou son dérivé étant ajouté au mélange réactionnel par addition dosée de manière que la concentration de 1,8-diène de formule I ou de son dérivé dans le mélange réactionnel n'excède pas 1 mole/l.

2. Procédé selon la revendication 1, dans lequel le mélange réactionnel contient en outre un agent d'entraînement.

3. Procédé selon la revendication 1 ou 2, dans lequel on utilise comme catalyseur pour la métathèse cyclisante un complexe ruthénium-carbène.

4. Procédé selon l'une quelconque des revendications 1 à 3, dans lequel, dans la métathèse cyclisante du 1,8-diène de formule I ou de son dérivé, outre le cycloheptène ou son dérivé on isole également en tant que produit de couplage du procédé une oléfine formée de formule V 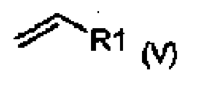 et/ou son produit de dimérisation, l'oléfine de formule VI

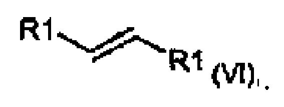

5. Procédé selon l'une quelconque des revendications 1 à 4, dans lequel on produit le 1,8-diène de formule I ou son dérivé par décarbonylation catalytique ou décarboxylation oxydante d'un acide carboxylique ou d'un dérivé d'acide carboxylique de formule VII 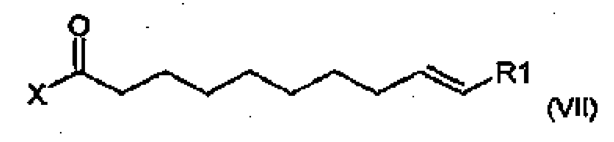 ou d'un dérivé de celui-ci, X étant un groupe partant.

6. Procédé selon la revendication 5, dans lequel le groupe partant X est choisi dans le groupe constitué par un atome d'hydrogène, un halogénure, le groupe OH et un groupe acyle.

7. Procédé selon l'une quelconque des revendications 1 à 6, dans lequel avant l'utilisation dans la réaction de métathèse cyclisante on purifie le 1,8-diène de formule I ou son dérivé.

8. Procédé selon l'une quelconque des revendications 1 à 7, dans lequel le 1,8-diène de formule I est l'heptadéca-1,8-diène, et dans lequel dans le cas des revendications 5 à 7 l'acide carboxylique ou le dérivé d'acide carboxylique de formule VII est l'acide oléique ou un dérivé d'acide oléique.

9. Procédé pour la production de cycloheptanone ou d'un dérivé de celle-ci par oxydation de cycloheptène ou d'un dérivé de celui-ci, qui est produit selon l'une quelconque des revendications 1 à 8, le dérivé étant le composé correspondant qui est substitué sur le cycle cycloheptène par un ou plusieurs radicaux R2, et R2 étant un radical choisi dans le groupe constitué par un groupe alkyle ayant de 1 à 4 atomes de carbone, un groupe alcoxy ayant de 1 à 4 atomes de carbone, le groupe phényle, un halogénure, le groupe amino, le groupe hydroxy et le groupe sulfo.

10. Procédé pour la production de cycloheptylamine ou d'un dérivé de celle-ci par conversion de cycloheptanone ou d'un dérivé de celle-ci, produit(e) selon la revendication 9, le dérivé étant le composé correspondant qui est substitué sur le cycle cycloheptène par un ou plusieurs radicaux R2, et R2 étant un radical choisi dans le groupe constitué par un groupe alkyle ayant de 1 à 4 atomes de carbone, un groupe alcoxy ayant de 1 à 4 atomes de carbone, le groupe phényle, un halogénure, le groupe amino, le groupe hydroxy et le groupe sulfo.

11. Procédé pour la production de cycloheptane-carbaldéhyde ou d'un dérivé de celui-ci par conversion de cycloheptène ou d'un dérivé de celui-ci, qui est produit selon l'une quelconque des revendications 1 à 8, le dérivé étant le composé correspondant qui est substitué sur le cycle cycloheptène par un ou plusieurs radicaux R2, et R2 étant un radical choisi dans le groupe constitué par un groupe alkyle ayant de 1 à 4 atomes de carbone, un groupe alcoxy ayant de 1 à 4 atomes de carbone, le groupe phényle, un halogénure, le groupe amino, le groupe hydroxy et le groupe sulfo.

12. Procédé pour la production d'acide cycloheptanecarboxylique ou d'un dérivé de celui-ci par conversion de cycloheptène ou d'un dérivé de celui-ci, qui est produit selon l'une quelconque des revendications 1 à 8, le dérivé étant le composé correspondant qui est substitué sur le cycle cycloheptène par un ou plusieurs radicaux R2, et R2 étant un radical choisi dans le groupe constitué par un groupe alkyle ayant de 1 à 4 atomes de carbone, un groupe alcoxy ayant de 1 à 4 atomes de carbone, le groupe phényle, un halogénure, le groupe amino, le groupe hydroxy et le groupe sulfo.

13. Procédé pour la production d'acide cycloheptanecarboxylique ou d'un dérivé de celui-ci par oxydation de cycloheptanecarbaldéhyde ou d'un dérivé de celui-ci, produit selon la revendication 11, le dérivé étant le composé correspondant qui est substitué sur le cycle cycloheptène par un ou plusieurs radicaux R2, et R2 étant un radical choisi dans le groupe constitué par un groupe alkyle ayant de 1 à 4 atomes de carbone, un groupe alcoxy ayant de 1 à 4 atomes de carbone, le groupe phényle, un halogénure, le groupe amino, le groupe hydroxy et le groupe sulfo.

14. Procédé pour la production de chlorure de cycloheptanecarbonyle ou d'un dérivé de celui-ci par conversion d'acide cycloheptanecarboxylique ou d'un dérivé de celui-ci, produit selon la revendications 12 ou 13, le dérivé étant le composé correspondant qui est substitué sur le cycle cycloheptène par un ou plusieurs radicaux R2, et R2 étant un radical choisi dans le groupe constitué par un groupe alkyle ayant de 1 à 4 atomes de carbone, un groupe alcoxy ayant de 1 à 4 atomes de carbone, le groupe phényle, un halogénure, le groupe amino, le groupe hydroxy et le groupe sulfo.
